# FASCICULE DE BREVET EUROPEEN

(11) **EP 0 895 467 B1**
(45) Date de publication et mention de la délivrance du brevet: **22.05.2002**
(21) Numéro de dépôt: 97907126.3
(22) Date de dépôt: 25.02.1997
(51) Int. Cl.: A61K 7/06

(54) **UTILISATION EN COSMETIQUE DE COPOLYMERES A SQUELETTE HYDROPHILE ET RIGIDE, GREFFES PAR DES MACROMONOMERES HYDROPHOBES ET FLEXIBLES; COMPOSITIONS MISES EN OEUVRE**
KOSMETISCHE VERWENDUNG VON KOPOLYMEREN MIT STARRER, HYDROPHILER HAUPTKETTE UND DARAN GEPFROPFTEN FLEXIBLEN HYDROPHOBEN MAKROMONOMEREN, SOWIE ZUSAMMENSETZUNGEN
COSMETIC USE OF COPOLYMERS WITH A RIGID HYDROPHILIC BACKBONE GRAFTED BY FLEXIBLE HYDROPHOBIC MACROMONOMERS, AND COMPOSITIONS THEREFOR

(30) Priorité: 27.03.1996 FR 9603814
(43) Date de publication de la demande: 10.02.1999
(73) Titulaire: L'OREAL, 75008 Paris (FR)
(72) Inventeur: SEBAG, Henri, F-75016 Paris (FR); MOUGIN, Nathalie, F-75011 Paris (FR); LION, Bertrand, F-93190 Livry-Gargan (FR); MONDET, Jean, F-93600 Aulnay-sous-Bois (FR)
(74) Mandataire: Dodin, Catherine
(86) Numéro de dépôt international: FR9700335
(87) Numéro de publication internationale: WO9735541

(56) Documents cités:
- EP-A- 0 320 218
- WO-A-92/16187
- WO-A-96/00562
- US-A- 4 496 708
- US-I5- B 464 491

## Description

La présente invention a trait à l'utilisation de copolymères à squelette, rigide et hydrophile, obtenus par polymérisation radicalaire ou polycondensation et greffés par des macromonomères, flexibles et hydrophobes dans et pour la préparation de compositions cosmétiques ou dermatologiques ainsi que les compositions mises en oeuvre.

Pour de nombreuses applications cosmétiques, notamment celles destinées au traitement et au soin des cheveux, de la peau ou des cils, on utilise des polymères susceptibles, après application sur le support à traiter et séchage, de former un dépôt ayant des propriétés mécaniques et des propriétés d'adhésion. On recherche dans cette optique des polymères à caractère hydrophile pour s'éliminer facilement sous l'action d'une solution aqueuse de tensioactifs. On recherche parallèlement à obtenir un dépôt hydrophobe en surface pour, d'une part, résister à l'humidité environnante notamment un dépôt non-hydroscopique au toucher, un dépôt résistant à la pluie (applications capillaires ou pour la peau) ou un dépôt résistant au liquide lacrymal (mascaras). D'autre part, on recherche un dépôt hydrophobe pour apporter des propriétés cosmétiques telles que la douceur au toucher généralement conférés par les substances hydrophobes en cosmétique.

Pour réunir toutes ces caractéristiques, on mélange en général des polymères d'hydrophilie et d'hydrophobicité différentes ou bien on associe à un polymère hydrophile une substance hydrophobe. Il est généralement difficile de contrôler parfaitement la stratification de ces mélanges après séchage du dépôt pour obtenir à la fois de bonnes propriétés mécaniques et d'adhésion et une surface hydrophobe.

Un autre problème relatif aux polymères de dépôt se pose dans le domaine du capillaire, en particulier dans le cadre des produits pour le maintien ou la fixation des cheveux. En effet, il est souvent difficile d'adapter les propriétés du polymère devant se déposer pour obtenir à la fois une bonne fixation de la chevelure, une bonne tenue de cette fixation et une élimination facile au peignage ou au brossage. En effet, pour réaliser une bonne fixation des cheveux, on plastifie le polymère fixateur pour ramener sa température de transition de phase à la température ambiante. On obtient alors dans ces conditions une élimination difficile au brossage ou au peignage.

On connaît, par le document EP-A-0320218, des copolymères adhésifs ayant une température de transition vitreuse basse, de l'ordre de 0-80°C. Ces copolymères trouvent une application dans le domaine cosmétique, notamment dans des compositions capillaires.

L'un des objectifs de la présente invention est donc d'utiliser dans des compositions cosmétiques ou dermatologiques des polymères ayant des propriétés filmogènes satisfaisantes, qui soient hydrophobes en surface et pouvant facilement s'éliminer sous l'action d'une solution aqueuse de tensioactifs.

Un autre objectif de l'invention est d'utiliser dans et pour la préparation de compositions capillaires de coiffage des polymères ayant à la fois des caractéristiques de fixation importantes résistant bien à de faibles sollicitations mécaniques et une bonne rigidité sans qu'il soit nécessaire d'ajouter un plastifiant ou du moins avec de faibles quantités de plastifiant, pour s'éliminer ensuite très facilement au brossage ou au peignage.

La Demanderesse a découvert de manière surprenante que ces objectifs pouvaient être atteints en utilisant dans et pour la préparation de compositions cosmétiques ou dermatologiques, des copolymères greffés particuliers dont le squelette est hydrophile et rigide, constitué d'un copolymère obtenu par polymérisation radicalaire ou par polycondensation et comportant sur la chaîne du squelette au moins un greffon macromonomère flexible et hydrophobe.

La présente invention a pour objet l'utilisation pour la préparation de compositions cosmétiques ou dermatologiques, d'un copolymère greffé dont le squelette (S) est constitué d'un copolymère hydrophile, de température de transition vitreuse Tg supérieure à 25°C, obtenu par polymérisation radicalaire ou par polycondensation et comportant sur la chaîne du squelette (S) au moins un greffon constitué d'un macromonomère (M) hydrophobe et de température de transition vitreuse T'g inférieure à 25°C.

La présente invention concerne également des compositions cosmétiques ou dermatologiques contenant dans un milieu cosmétiquement acceptable au moins un copolymère greffé dont le squelette est constitué d'un copolymère (S) hydrophile, de température de transition vitreuse Tg supérieure à 25°C, obtenu par polymérisation radicalaire ou par polycondensation et comportant sur la chaîne du squelette au moins un greffon constitué d'un macromonomère (M) hydrophobe et de température de transition vitreuse T'g inférieure à 25°C.

D'autres objets apparaîtront à la lumière de la description et des exemples qui suivent.

On entend par «copolymère hydrophile» dans tout le texte de la description, tout copolymère soluble ou dispersible dans l'eau, les alcools inférieurs (en C₁-C₄) ou les mélanges d'eau et d'alcool(s) inférieur(s).

On entend par «polymère hydrophobe» dans tout le texte de la description, tout polymère insoluble dans l'eau, les alcools inférieurs (en C₁-C₄) ou les mélanges d'eau et d'alcool(s) inférieur(s).

On entend par «macromonomère» dans tout le texte de la description, tout oligomère comportant sur une seule extrémité, soit un groupe à insaturation éthylénique susceptible de polymériser par voie radicalaire avec les monomères constituant le squelette (S) du copolymère de l'invention et de se greffer sur la chaîne polymérique du squelette (S) ; soit un groupe fonctionnel réactif susceptible de réagir avec les monomères (A) et (B) du squelette (S) ou bien avec le squelette (S) préalablement formé.

Les macromonomères (M) greffés par liaison covalente sur la chaîne polymérique du squelette (S) des copolymères de l'invention, sont choisis préférentiellement parmi les macromonomères hydrocarbonés, hydrofluorocarbonés ou fluorocarbonés ayant une température de transition vitreuse T'g inférieure à 25°C.

Les macromonomères (M) ont une température de transition vitreuse T'g de préférence inférieure ou égale à 10°C et plus particulièrement inférieure ou égale à 0°C.

Les macromonomères (M) sont de plus hydrophobes, c'est-à-dire insolubles dans l'eau et ont une tension de surface généralement inférieure ou égale à 40 dyne/cm à 20°C.

Ils présentent de préférence un poids moléculaire moyen mesuré en sommet de pic par chromatographie d'exclusion stérique allant de 200 à 20 000.

Parmi les macromonomères (M) greffés sur les copolymères de l'invention, on peut citer :
(a) les polymères et les copolymères d'acrylate ou de méthacrylate d'alkyle linéaire ou ramifié en C₂-C₁₈, de T'g inférieure à 25°C et présentant un groupe terminal choisi parmi vinyle, allyle, méthallyle, (méth)acryloyle, éthacryloyle, vinylbenzoyle, vinylbenzyle, alcényle en C₁-C₄, cycloalcényle en C₁-C₆, ou une fonction réactive terminale susceptible d'interagir avec le squelette (S) ou les monomères le constituant (telle que -OH, -NH₂, -COOH, anhydride) ou une fonction réactive terminale susceptible de participer à une polycondensation (telle que diol, diamine, diacide carboxylique), parmi lesquels on peut citer en particulier : les macromonoméres de poly(acrylate de butyle) à extrémité monométhacrylate tels que les produits vendus sous le nom par la société TOA GOSEI ; les macromonomères de poly(acrylate de butyle) à extrémité monométhacrylate ; les macromonomères de poly(acrylate d'éthyl-2 hexyle) à extrémité monoacrylate ou monométhacrylate ; les macromonomères de poly(acrylate de dodécyle) ou de poly(méthacrylate de dodécyle) ; les macromonomères de poly (acrylate de stéaryle) ou de poly (méthacrylate de stéaryle) ;
(b) les polyoléfines de T'g inférieure à 25°C et présentant un groupe terminal à insaturation éthylénique ou une fonction réactive terminale susceptible d'interagir avec le squelette (S) ou les monomères le constituant ou une fonction réactive terminale susceptible de participer à une polycondensation, parmi lesquels on peut citer en particulier :
   les macromonomères de polyéthylène, les macromonomères de polypropylène, les macromonomères de copolymère polyéthylène/polypropylène, les macromonomères de copolymère polyéthylène/polybutylène, les macromonomères de polyisobutylène ; les macromonomères de polybutadiène ; les macromonomères de polyisoprène ; les macromonomères de polyoléfine à chaîne très courte comme par exemple les macromonomères de polybutadiène hydrogéné ou de polyisoprène hydrogéné ne comportant que 3 ou 4 motifs répétitifs, et plus particulièrement l'acrylate ou le méthacrylate de phytol (tétraméthyl 3, 7 11, 15-2 hexadécène-1-ol).
(c) les polymères vinyliques de T'g inférieure à 25°C et présentant un groupe terminal à insaturation éthylénique ou une fonction réactive terminale susceptible d'interagir avec le squelette (S) ou les monomères le constituant ou une fonction réactive terminale susceptible de participer à une polycondensation ;
(d) les polymères ou copolymères de monomères fluorés ou perfluorés, de T'g inférieure à 25°C et présentant un groupe terminal à insaturation éthylénique ou une fonction réactive terminale susceptible d'interagir avec le squelette (S) ou les monomères ie constituant ou une fonction réactive terminale susceptible de participer à une polycondensation, parmi lesquels on peut citer en particulier les homopolymères ou copolymères de (méth)acrylate de perfluoroalkyle ;
(e) les polyesters de T'g inférieure à 25°C et présentant un groupe terminal à insaturation éthylénique ou une fonction réactive terminale susceptible d'interagir avec le squelette (S) ou les monomères le constituant ou une fonction réactive terminale susceptible de participer à une polycondensation, parmi lesquels on peut citer en particulier les polyesters aliphatiques constitués de longues séquences carbonées comme les polyesters d'acide 12-hydroxystéarique, les polysébaçates de diols aliphatiques constitués de longues séquences carbonées (par exemple hexanediol).

Les macromonomères (M) sont présents dans la composition des copolymères de l'invention dans une proportion allant de préférence de 1 à 60% en poids par rapport au poids total du copolymère greffé.

Les copolymères greffés conformes à la présente invention, présentent de préférence un poids moléculaire moyen mesuré en sommet de pic par chromatographie d'exclusion stérique allant de 10 000 à 5 000 000.

Ils sont en général hydrophiles à savoir solubles ou dispersibles dans les milieux aqueux, les milieux alcooliques ou hydroalcooliques à base d'alcools inférieurs. Ils peuvent être non-ioniques, anioniques, cationiques ou amphotères ; les groupes ioniques étant situés préférentiellement dans la structure du squelette (S) pour apporter l'hydrophilie.

Le squelette (S) des copolymères de l'invention ont une température de transition de phase Tg supérieure à 25°C et de préférence supérieure ou égale à 35°C.

Le squelette (S) des copolymères de l'invention est constitué d'un copolymère obtenu par polymérisation radicalaire ou par polycondensation.

Le squelette (S) obtenu par voie radicalaire, résulte, de préférence, de la polymérisation :
(a) d'au moins un monomère ou un mélange de monomères (A) à insaturation éthylénique, et
(b) d'au moins un monomère ou un mélange de monomères (B) polaires et hydrophiles, à insaturation éthylénique ; les monomères (A) et (B) étant choisis de telle sorte que la température de transition de phase Tg du squelette (S) soit supérieure à 25°C.

Les monomères du type (A) sont choisis par exemple dans le groupe constitué par :
- les esters ou les amides acryliques ou méthacryliques obtenus à partir d'alcools aliphatiques, linéaires, ramifiés ou cycliques et/ou d'alcools aromatiques, de préférence en C₁-C₄ tel que le (méth)acrylate de méthyle, le (méth)acrylate d'éthyle, le (méth)acrylate de propyle, le (méth)acrylate de butyle, le (méth)acrylate d'isobutyle, le (méth)acrylate de tertio-butyle, le tertio-butyl acrylamide ;
- les esters ou amides vinyliques, allyliques ou méthallyliques obtenus à partir d'alcools aliphatiques, linéaires, ramifiés ou cycliques et/ou d'alcools aromatiques, de préférence en C₁-C₆, tels que l'acétate de vinyle, le propionate de vinyle, le benzoate de vinyle, le tertio-butyl benzoate de vinyle ;
- les oléfines tels que l'éthylène, le propylène, le styrène ou le styrène substitué ;
- les monomères acryliques ou vinyliques fluorés ou perfluorés ;
- leurs mélanges.

Les monomères du type (B) de l'invention sont choisis parmi les monomères hydrophiles et polaires à insaturation éthylénique anioniques, cationiques, amphotères ou non-ioniques ou leurs mélanges.

Parmi les monomères (B) anioniques, on peut citer :
- les monomères comportant au moins une fonction acide, sous forme libre ou bien sous forme partiellement ou totalement neutralisée tels que les mono acides carboxyliques comme les acides acrylique, méthacrylique, crotonique ; les diacides carboxyliques ou les anhydrides d'acide ainsi que leurs monoesters ou monoamides comme l'anhydride maléique sous forme de diacide, de monoester ou monoamide, l'acide itaconique ;
- les monomères comportant au moins une fonction acide sulfonique, sous forme libre ou bien sous forme partiellement ou totalement neutralisée tels que l'acide vinyl- ou styrène sulfonique, l'acide acrylamido-2 méthylpropane-2 sulfonique ;
- les monomères comportant au moins une fonction acide phosphorique ou phosphonique, sous forme libre ou bien sous forme partiellement ou totalement neutralisée.

Les monomères anioniques (B) sont de préférence partiellement ou totalement neutralisés par un composé monobasique telle qu'une base minérale comme la soude ou la potasse, ou un aminoalcool par exemple pris dans le groupe constitué par l'amino-2 méthyl-2 propanol-1 (AMP), la triéthanolamine, la triisopropanolamine (TIPA), la monoéthanolamine, la tri[(hydroxy-2) propyl-1 amine, l'amino-2 méthyl-2 propanediol-1,3 (AMPD), l'amino-2 hydroxyméthyl-2 propanediol-1,3.

Parmi les monomères (B) cationiques, on peut citer :
- les monomères comportant une fonction amine sous forme libre ou bien partiellement ou totalement neutralisée ou bien partiellement ou totalement quaternisée tels que le (méth)acrylate de diméthylaminoéthyle, le diméthylaminoéthyl méthacrylamide, la vinylamine, la vinylpyridine, le chlorure de diallyldiméthylammonium.

Les monomères cationiques (B) sont de préférence partiellement ou totalement neutralisés par un acide organique minéral ou organique tel que les acides chlorhydrique, acétique, lactique ou glycolique ou bien partiellement ou totalement quaternisés par un halogénure d'alkyle, de cycloalkyle, d'aryle ou un dialkylsulfate (diméthyl- ou diéthylsulfate).

Parmi les monomères (B) amphotères, on peut citer les carboxybétaïnes ou les sulfobétaïnes obtenues par quaternisation partielle ou totale de monomères à insaturation éthylénique comportant une fonction amine par des sels de sodium d'acide carboxylique à halogénure mobile (chloroacétate de sodium) ou par des sultones cycliques (propane sultone).

Parmi les monomères (B) non-ioniques, on peut citer :
- les (méth)acrylate d'hydroxyalkyle en C₁-C₄ tels que (méth)acrylate de 2-hydroxy éthyle, (méth)acrylate de 2-hydroxy propyle,
- les acrylamides tels que acrylamide, méthacrylamide, les dialkyl(C₁-C₄) (méth) acrylamides ;
- la N-vinylpyrrolidone ;
- les (méth)acrylates d'éthylèneglycol, de diéthylèneglycol, de polyéthylèneglycol à extrémité hydroxyle ou éther.

Le squelette (S) obtenu par polycondensation résulte, de préférence, de la réaction:
(a) d'au moins un monomère ou un mélange de monomères (A') polycondensable et éventuellement
(b) d'un monomère ou un mélange de monomères (B') polycondensable avec le ou les monomères (A') portant au moins un groupe fonctionnel hydrophile apportant la solubilité ou la dispersibilité dans l'eau, les milieux alcooliques ou les milieux hydroalcooliques ; les monomères (A') et (B') étant choisis de telle sorte que la température de transition de phase Tg du squelette (S) soit supérieure à 25°C.

Les squelettes (S) du type polycondensat sont choisis par exemple parmi les polyesters, les polyamides, les polyuréthanes ou les polyesteramides.

Les copolymères greffés conformes à l'invention peuvent être obtenus par copolymérisation directe radicalaire de monomères (A) et (B) tels que définis ci-dessus constituant le squelette (S) et d'un macromonomère (M) présentant sur une seule extrémité un groupe à insaturation éthylénique copolymérisable avec les monomères (A) et (B).

La polymérisation directe radicalaire peut être faite alors en solution dans un solvant commun ou un mélange de solvants communs. Elle peut également être effectuée en milieu hétérogène, en particulier en suspension ou en émulsion dans l'eau ; le macromonomère étant dissous dans le mélange avec les monomères (A) et (B) tels que définis ci-dessus.
Lorsque le squelette (S) est un polycondensat tel qu'un polyester, un polyamide, un polyuréthane ou un polyesteramide, les copolymères greffés conformes à l'invention peuvent être obtenus par polycondensation directe de monomères (A') et (B') tels que définis ci-dessus constituant le squelette (S) et d'un macromonomère (M) présentant sur une seule extrémité deux fonctions réactives terminales (par exemple diol, diamine, diacide carboxylique, anhydride d'acide) susceptible de polycondenser avec les monomères (A') et (B').

La polycondensation directe peut être réalisée en solution, en dispersion ou en milieu fondu selon une réaction du type estérification, amidification, transestérification ou transamidification.

Enfin, les copolymères greffés conformes à l'invention peuvent être aussi obtenus en faisant réagir le copolymère du squelette (S), préalablement synthétisé, avec un macromonomère (M) présentant une fonction terminale réactive appropriée susceptible d'interagir avec le squelette (S) de préférence monofonctionnelle (amine, alcool, acide carboxylique, anhydride, époxy...). La réaction est généralement effectuée en solution ou dans un milieu fondu.

Les compositions cosmétiques et dermatologiques selon l'invention contiennent donc dans un support cosmétiquement acceptable les polymères tels que décrits ci-dessous, pour des applications aussi variées que celles rencontrées par exemple dans le domaine du capillaire, du maquillage ou bien encore des soins de la peau, ou de tout autre domaine cosmétique dans lequel l'utilisation d'une substance filmogène est désirable ou recherchée.

Les copolymères greffés selon l'invention peuvent être utilisés seuls comme agent filmogène ou bien comme additif à des agents filmogènes conventionnels dans et pour la préparation de compositions cosmétiques ou dermatologiques.

Parmi les applications préférentiellement visées par la présente invention, on peut plus particulièrement mentionner :
- le domaine des produits capillaires (lavage, soin ou beauté des cheveux), où les compositions selon l'invention, peuvent se présenter sous forme d'aérosols, de mousse, de shampooings, d'après-shampooings, de lotions ou de gels coiffants ou traitants, laques ou lotions de mise en forme ou de mise en plis ou encore de fixation
- le domaine des produits de maquillage, en particulier pour le maquillage des ongles, des cils ou des lèvres, où les compositions selon l'invention peuvent se présenter sous forme de vernis à ongle ; de mascaras ou de eye-liners ; de rouges à lèvres.
- dans le domaine des produits de soin de la peau (crèmes, laits, lotions, masques, sérums, produits solaires).

La concentration en copolymère greffé dans les compositions cosmétiques ou dermatologiques de l'invention est généralement comprise entre 0,1 et 50%, et de préférence entre 1 et 30% en poids total de la composition. Elle varie selon l'application cosmétique ou dermatologique envisagée.

Dans le cas des vernis à ongles, cette proportion est en général supérieure ou égale à 30% en poids lorsque le copolymère de l'invention est utilisé seul comme agent filmogène.

Le support cosmétiquement acceptable des compositions selon l'invention est de préférence constitué d'eau, d'un ou plusieurs solvants organiques cosmétiquement acceptables ou bien d'un mélange d'eau et d'un ou plusieurs solvants organiques cosmétiquement acceptables.

Parmi ces solvants organiques, on utilise plus particulièrement les alcools inférieurs en C₁-C₄ tels que l'éthanol.

Les copolymères greffés selon l'invention sont dissous ou en dispersion dans le support des compositions de l'invention.

Les compositions peuvent en outre, et bien entendu, contenir divers adjuvants destinés à la rendre acceptable dans une application cosmétique particulière.

Les compositions selon l'invention peuvent contenir des additifs cosmétiques conventionnels choisis parmi les corps gras tels que les huiles minérales, végétales animales ou de synthèse, les cires animales, fossiles, végétales, minérales ou de synthèse, des solvants organiques, des agents épaississants, des adoucissants, des agents anti-mousse, des agents hydratants, des humectants, des agents traitants (agents anti-chute, anti-pelliculaire,...), des antiperspirants, des agents alcanisants, des filtres solaires UV-A ou UV-B ou à bande large, des colorants, des pigments, des parfums, des plastifiants, des conservateurs, des polymères organiques anioniques, non-ioniques ou amphotères compatibles avec les copolymères greffés de l'invention et des agents propulseurs lorsque les compositions se présentent sous forme aérosol.

Bien entendu, l'homme de l'art veillera à choisir le ou les éventuels composés complémentaires mentionnés ci-avant, de manière telle que les propriétés avantageuses attachées intrinsèquement aux compositions selon l'invention ne soient pas, ou substantiellement pas, altérées par la ou les adjonctions envisagées.

L'invention a également pour objet un procédé de traitement cosmétique des matières kératiniques telles que la peau, les cheveux, le cuir chevelu, les cils, les sourcils, les ongles, les lèvres, caractérisé en ce qu'il consiste à appliquer sur ces dernières une composition telle que définie ci-dessus.

Les exemples qui suivent servent à illustrer l'invention sans toutefois présenter un caractère limitatif.

### EXEMPLES DE 1 A 6 DE PREPARATION

### EXEMPLE 1 : Préparation d'un télomère poly(acrylate d'éthyl-2 hexyle) à terminaison OH et de poids moléculaire 1150

Dans un réacteur avec barbotage d'azote, réfrigérant, agitation mécanique centrale, thermomètre, on introduit successivement : 52,1g d'acrylate d'éthyl-2 hexyle, 100g de tétrahydrofurane, 3,9g de mercaptoéthanol et 0,5g d'azobisisobutyronitrile. On homogénéise sous agitation à température ambiante avec barbotage d'azote. On chauffe ensuite sous agitation à 66°C en maintenant le barbotage d'azote et on laisse réagir dans ces conditions pendant 24 heures. En fin de synthèse, on ramène à température ambiante, on évapore le solvant à pression atmosphérique dans le réacteur, puis on distille les restes de mercaptoéthanol en établissant un vide de 10⁻² millibars. On obtient ainsi le télomère sous forme d'huile.

L'indice d'hydroxyle final est de 48,9 ce qui correspond à un poids moléculaire de 1150 pour une fonctionnalité de 1.

La caractérisation de la distribution des poids moléculaires par chromatographie d'exclusion stérique dans le tétrahydrofurane donne 4 pics principaux à 1720, 700, 460 et 170.

### EXEMPLE 2 : Préparation d'un macromonomère (M) à terminaison acrylate obtenu à partir du télomère de l'exemple 1

Dans un réacteur de 500ml à agitation centrale, thermomètre, réfrigérant, on introduit 30g de télomère de l'exemple 1 (0,0262 moles), 3,2g triéthylamine et 60g de toluène. Au-dessus du réacteur et contenu dans une ampoule à introduction, on place 2,85g (0,0312 moles) de chlorure d'acryloyle dans 10g de toluène. On refroidit le réacteur à 5°C et on introduit goutte à goutte la solution de chlorure d'acryloyle en maintenant la température inférieure à 10°C. Une fois l'ajout effectué, on laisse revenir sous agitation à température ambiante et on maintient la réaction pendant 18 heures.

On filtre la solution obtenue sur fritté pour éliminer le précipité de chlorhydrate de triéthylamine. Le filtrat obtenu est dilué par 500ml de chlorure de méthylène et extrait deux fois à l'eau. Après extraction, la solution est débarrassée du solvant par évaporation sous vide à l'évaporateur rotatif.

On obtient ainsi un rendement global de 80% (étape de télomérisation de l'exemple 1 et étape de fonctionnalisation). Le produit se présente sous forme d'huile.

La caractérisation de la distribution des poids moléculaires par chromatographie d'exclusion stérique dans le tétrahydrofurane donne 4 pics principaux à 1900, 880, 580 et 350.

La température de transition vitreuse T'g théorique de ce macromonomère est inférieure à - 50°C d'après le POLYMER HANDBOOK 3ème édition, Wiley Interscience.

### EXEMPLE 3 : Préparation d'un télomère poly(acrylate d'éthyl-2 hexyle) à terminaison OH et de poids moléculaire 4650

On opère dans les mêmes conditions que l'exemple 1 en utilisant 52,1g d'acrylate d'éthyl-2 hexyle, 100g de tétrahydrofurane, 1g de mercaptoéthanol et 0,45g d'azobisisobutyronitrile.

L'indice d'hydroxyle final est de 72 ce qui correspond à un poids moléculaire de 4650 pour une fonctionnalité de 1.

### EXEMPLE 4 : Préparation d'un macromonomère (M) à terminaison acrylate obtenu à partir du télomère de l'exemple 3

On opère dans les mêmes conditions que l'exemple 2 en utilisant :
- dans le réacteur : 30g de télomère de l'exemple 3 (0,0066 moles), 0,78g triéthylamine et 70g de toluène ;
- dans l'ampoule à introduction, 0,7g (0,0079 moles) de chlorure d'acryloyle dans 10g de toluène.

On obtient ainsi un rendement global de 80% (étape de télomérisation de l'exemple 3 et étape de fonctionnalisation). Le produit se présente sous forme d'huile.

La caractérisation de la distribution des poids moléculaires par chromatographie d'exclusion stérique donne 1 seul pic principal correspondant à un poids moléculaire de 4300.

La température de transition vitreuse T'g théorique de ce macromonomère est inférieure à -50°C d'après le POLYMER HANDBOOK 3ème édition, Wiley Interscience.

### EXEMPLE 5 : Préparation d'un copolymère acrylique greffé à partir du macromonomère (M) de l'exemple 2

On réalise le copolymère greffé acrylique à partir de la composition suivante :
- Acrylate de tertio-butyle 60% en poids
- Acide acrylique 20% en poids
- Macromonomère (M) de l'exemple 2 20% en poids

Dans un réacteur avec agitation centrale, réfrigérant, thermomètre, barbotage d'azote, on introduit 100g du mélange de monomères décrit ci-dessus, 100g d'éthanol et 1ml de tertio-butyl peroxy 2-éthyl hexanoate à 97% (vendu par la société AKZO sous le nom TRIGONOX 21 S). On agite à température ambiante sous barbotage d'azote pour homogénéiser l'ensemble. On porte ensuite au reflux (78°C) sous agitation et barbotage d'azote. On laisse réagir dans ces conditions pendant 18 heures. En fin de synthèse, on revient à température ambiante, on dilue par 50 ml environ d'acétate d'éthyle et on purifie le polymère par précipitation de la solution dans 8 I d'éther de pétrole. On sèche le précipité sous vide à température de 50°C jusqu'à poids constant.

Le rendement est de 80%. L'indice d'acide est de 168,5.

La caractérisation de la distribution des poids moléculaires par chromatographie d'exclusion stérique donne 1 seul pic principal correspondant à un poids moléculaire de 101 000.

La température de transition vitreuse Tg du squelette, mesurée par DSC (calorimétrie différentielle) est de 73°C.

### EXEMPLE 6 : Préparation d'un copolymère acrylique greffé à partir du macromonomère (M) de l'exemple 4

On réalise le copolymère greffé acrylique à partir de la composition suivante:
- Acrylate de tertio-butyle 60% en poids
- Acide acrylique 20% en poids
- Macromonomère (M) de l'exemple 4 20% en poids
On opère dans les même conditions que l'exemple 5.

Le rendement est de 85%. L'indice d'acide est de 180.

La caractérisation de la distribution des poids moléculaires par chromatographie d'exclusion stérique donne 1 seul pic principal correspondant à un poids moléculaire de 115 400.

La température de transition vitreuse Tg du squelette, mesurée par DSC (calorimétrie différentielle) est de 90°C.

### EXEMPLE 7 : Préparation d'un macromonomère (P) polyisobutylène possédant une terminaison acrylamido

On part d'un produit commercial vendu sous le nom KEROCOM PIBA par la société qui est un macromonomère polyisobutylène à extrémité amine primaire dont la masse mesurée par chromatographie d'exclusion stérique sur colonnes de microstyragel avec le tétrahydrofuranne comme éluant et des étalons polystyrène est égale à 2000 en sommet de pic GPC. Pour obtenir un macromonomère polymérisable radicalairement, on fait réagir ce polyisobutylène à extrémité amino avec le chlorure d'acryloyle.

Dans un réacteur de 500ml à agitation mécanique centrale, thermomètre, réfrigérant, on introduit 50g de macromonomère KEROCOM PIBA (0,05 moles), 6,06 g de triéthylamine (0,06 moles) et 100g de toluène. On agite pour dissoudre les réactifs et on refroidit au bain de glace. Au-dessus du réacteur et contenu dans une ampoule à introduction, on place 5,43 g (0,06 moles) de chlorure d'acryloyle dans 20g de toluène. On ajoute cette solution goutte à goutte dans le réacteur sous agitation en maintenant la température du milieu réactionnel entre 0°C et 10°C. A la fin de l'ajout, on laisse remonter la température à 25°C et on maintien dans ces conditions pendant 18 heures.

On évapore le toluène du milieu réactionnel et on ajoute 300 g de dichlorométhane. On extrait cette solution par une solution aqueuse de NaCl à 100 g/l en utilisant 300 g de solution saline. L'extraction est faite en ampoule. On fait ainsi trois extractions successives dans les mêmes conditions. On récupère au final la phase organique que l'on sèche au Na₂SO₄. On distille ensuite le dichlorométhane à l'évaporateur pour obtenir le macromonomère à extrémité acrylamido.

Le rendement obtenu est 72%.

La masse moléculaire en sommet de pic GPC, mesurée par chromatographie d'exclusion stérique sur colonnes de microstyragel avec le tétrahydrofuranne comme éluant et des étalons polystyrène est de 2480.

### EXEMPLE 8 : Préparation d'un copolymère acrylique greffé à partir du macromonomère (P) de l'exemple 7

On réalise le copolymère greffé acrylique à partir de la composition suivante :
- Acrylate de tertio-butyle 70% en poids
- Acide acrylique 20% en poids
- Macromonomère (P) de l'exemple 7 10% en poids

Dans un réacteur de 1 litre avec agitation mécanique centrale, réfrigérant, thermomètre, barbotage d'azote, on introduit 100g du mélange de monomères décrit ci-dessus, 60g de tétrahydrofurane et 40 g de cyclohexane et 1ml de tertio-butyl peroxy 2-éthyl hexanoate à 97% (vendu par la société AKZO sous le nom TRIGONOX 21 S). On agite à température ambiante sous barbotage d'azote pour homogénéiser l'ensemble. On chauffe ensuite à 78°C sous agitation et barbotage d'azote. On laisse réagir dans ces conditions pendant 18 heures. En fin de synthèse, on revient à température ambiante, on dilue par 200 g environ d'acétate d'éthyle et on purifie le polymère par précipitation de la solution dans 6 I d'éther de pétrole. On sèche le précipité sous vide à température de 45°C jusqu'à poids constant.

Le rendement est de 90%. L'indice d'acide est de 167.

La caractérisation de la distribution des poids moléculaires par chromatographie d'exclusion stérique donne 1 seul pic principal correspondant à un poids moléculaire de 54 300.

La température de transition vitreuse Tg du squelette, mesurée par DSC (calorimétrie différentielle) est de 80°C.

### EXEMPLE 9 : Préparation d'un copolymère acrylique à greffons polyacrylate de butyle

On réalise le copolymère greffé acrylique à partir de la composition suivante :
- Acrylate de tertio-butyle 60% en poids
- Acide acrylique 20% en poids
- Macromonomère Polyacrylate de butyle de Tg -30 de poids moléculaire en nombre 6000 vendu sous le nom AB-6
   par TOA GOSEI 20% en poids

Dans un réacteur de 1 litre avec agitation mécanique centrale, réfrigérant, thermomètre, barbotage d'azote, on introduit 100g du mélange de monomères décrit ci-dessus, 100 g d'éthanol et 1ml de tertio-butyl peroxy 2-éthyl hexanoate à 97% (vendu par la société AKZO sous le nom TRIGONOX 21 S). On agite à température ambiante sous barbotage d'azote pour homogénéiser l'ensemble. On chauffe ensuite à 78°C sous agitation et barbotage d'azote. On laisse réagir dans ces conditions pendant 18 heures. En fin de synthèse, on revient à température ambiante, on dilue par 200 g environ d'acétate d'éthyle et on purifie le polymère par précipitation de la solution dans 6 I d'éther de pétrole. On sèche le précipité sous vide à température de 45°C jusqu'à poids constant.

Le rendement est de 85%. L'indice d'acide est de 162.

La caractérisation de la distribution des poids moléculaires par chromatographie d'exclusion stérique donne 1 seul pic principal correspondant à un poids moléculaire de 99 000.

La température de transition vitreuse Tg du squelette, mesurée par DSC (calorimétrie différentielle) est de 80°C.

### EXEMPLES A, B ET C DE COMPOSITION

### EXEMPLE A : Spray de coiffage en aérosol

### Composition A :

- Copolymère acrylique greffé de l'exemple 5 8% en poids M.A. (M.A.matière active)
- Amino-2 méthyl-2 propanol-1 pour neutralisation à 50% qsp
- Ethanol qsp 100% en poids

### Pressurisation :

- Composition A 37% en poids
- Diméthyléther 43% en poids
- Pentane 20% en poids

### EXEMPLE B : Spray de coiffage en aérosol

### Composition B:

- Copolymère acrylique greffé de l'exemple 6 5,4% en poids M.A.
- Amino-2 méthyl-2 propanol-1 pour neutralisation à 75% qsp
- Ethanol qsp 100% en poids

### Pressurisation :

- Composition B 37% en poids
- Diméthyléther 43% en poids
- Pentane 20% en poids

### EXEMPLE C : Spray de coiffage en flacon pompe

- Copolymère acrylique greffé de l'exemple 5 2% en poids M.A.
- Amino-2 méthyl-2 propanol-1 pour neutralisation à 50% qsp
- Ethanol qsp 100% en poids

Les trois compositions A, B et C, après application sur cheveux en finition de coiffure, apportent une bonne fixation de la chevelure avec une bonne facilité de démêlage et un toucher lisse et agréable après brossage.

### EXEMPLE D : Spray de coiffage en aérosol

### Composition D :

- Copolymère acrylique greffé de l'exemple 8 8% en poids M.A.
- Amino-2 méthyl-2 propanol-1 pour neutralisation à 50% qsp
- Ethanol qsp 100% en poids

### Pressurisation :

- Composition D 50% en poids
- Diméthyléther 50% en poids

### EXEMPLE E : Spray de coiffage en aérosol

### Composition E :

- Copolymère acrylique greffé de l'exemple 9 8% en poids M.A.
- Amino-2 méthyl-2 propanol-1 pour neutralisation à 50% qsp
- Ethanol qsp 100% en poids

### Pressurisation :

- Composition E 50% en poids
- Diméthyléther 50% en poids

Les deux compositions D et E, après application sur cheveux en finition de coiffure, apportent un bon pouvoir laquant sur les cheveux sans effet de poissage, une bonne facilité de démêlage et une bonne élimination aux shampooings.

## Revendications

1. Utilisation pour la préparation de compositions cosmétiques ou dermatologiques, d'ur copolymère greffé dont le squelette (S) est constitué d'un copolymère hydrophile, de température de transition vitreuse Tg supérieure à 25°C, obtenu par polymérisation radicalaire ou par polycondensation et comportant sur la chaîne du squelette au moins un greffon constitué d'un macromonomère (M) hydrophobe et de température de transition vitreuse T'g inférieure à 25°C.

2. Utilisation selon la revendication 1, selon laquelle les macromonomères (M) greffés sont choisis parmi les macromonomères hydrophobes hydrocarbonés, hydrofluorocarbonés ou fluorocarbonés ayant une température de transition de phase T'g inférieure à 25°C.

3. Utilisation selon la revendication 1 ou 2, selon laquelle les macromonomères (M) greffés ont une température de transition de phase T'g inférieure à 10°C et de préférence inférieure ou égale à 0°C.

4. Utilisation selon l'une quelconque des revendications 1 à 3, selon laquelle les macromonomères (M) greffés ont une tension de surface généralement inférieure ou égale à 40 dyne/cm à 20°C.

5. Utilisation selon l'une quelconque des revendications 1 à 4, selon laquelle les macromonomères (M) greffés ont un poids moléculaire moyen, mesuré en sommet de pic par chromatographie d'exclusion stérique allant de 200 à 20 000.

6. Utilisation selon l'une quelconque des revendications 1 à 5, selon laquelle les macromonomères (M) greffés sont choisis dans le groupe constitué par :
(a) les polymères et les copolymères d'acrylate ou de méthacrylate d'alkyle linéaire ou ramifié en C₂-C₁₈, de T'g inférieure à 25°C et présentant un groupe terminal choisi parmi vinyle, allyle, méthallyle, (méth)acryloyle, éthacryloyle, vinylbenzoyle, vinylbenzyle, alcényle en C₁-C₄, cycloalcényle en C₁-C₆, ou une fonction réactive terminale susceptible d'interagir avec le squelette (S) ou les monomères le constituant ou une fonction réactive terminale susceptible de participer à une polycondensation ;
(b) les polyoléfines de T'g inférieure à 25°C et présentant un groupe terminal à insaturation éthylénique ou une fonction réactive terminale susceptible d'interagir avec le squelette (S) ou les monomères le constituant ou une fonction réactive terminale susceptible de participer à une polycondensation ;
(c) les polymères vinyliques de T'g inférieure à 25°C et présentant un groupe terminal à insaturation éthylénique ou une fonction réactive terminale susceptible d'interagir avec le squelette (S) ou les monomères le constituant ou une fonction réactive terminale susceptible de participer à une polycondensation ;
(d) les polymères ou copolymères de monomères fluorés ou perfluorés, de T'g inférieure à 25°C et présentant un groupe terminal à insaturation éthylénique ou une fonction réactive terminale susceptible d'interagir avec le squelette (S) ou les monomères le constituant ou une fonction réactive terminale susceptible de participer à une polycondensation ;
(e) les polyesters de T'g inférieure à 25°C et présentant un groupe terminal à insaturation éthylénique ou une fonction réactive terminale susceptible d'interagir avec le squelette (S) ou les monomères le constituant ou une fonction réactive terminale susceptible de participer à une polycondensation.

7. Utilisation selon la revendication 6, selon laquelle les macromères sont choisis dans le groupe constitué par : les macromonomères de poly(acrylate de butyle) à extrémité monométhacrylate ; les macromonomères de poly(acrylate de butyle) à extrémité monométhacrylate ; les macromonomères de poly(acrylate d'éthyl-2 hexyle) à extrémité monoacrylate ou monométhacrylate ; les macromonomères de poly(acrylate de dodécyie) ou de poly(méthacrylate de dodécyle) ; les macromonomères de poly (acrylate de stéaryle) ou de poly (méthacrylate de stéaryle) ; les macromonomères de polyéthylène, les macromonomères de polypropylène, les macromonomères de copolymère polyéthylène/polypropylène ; les macromonomères de copolymère poly éthylènelpolybutylène ; les macromonomères de polybutadiène ; les macro monomères de polyisoprène ; les macromonomères de polybutadiène hydrogéné ou de polyisoprène hydrogéné ne comportant que 3 ou 4 motifs répétitifs et plus particulièrement l'acrylate ou le méthacrylate de phytol (tétraméthyl 3, 7 11, 15-2 hexadécène-1-ol) ; les homopolymères ou copolymères de (méth)acrylate de perfluroalkyle ; les polyesters aliphatiques constitués de longues séquences carbonées.

8. Utilisation selon l'une quelconque des revendications 1 à 7, selon laquelle les macromonomères (M) greffés sont présents dans la composition des copolymères de l'invention dans une proportion allant de 1 à 60% en poids par rapport au poids total du copolymère greffé.

9. Utilisation selon l'une quelconque des revendications 1 à 7, **caractérisée par le fait que** le copolymère greffé présente un poids moléculaire moyen mesuré en sommet de pic par chromatographie d'exclusion stérique allant de 10 000 à 5 000 000.

10. Utilisation selon l'une quelconque des revendications 1 à 8, **caractérisée par le fait que** le squelette (S) des copolymères de l'invention a une température de transition de phase Tg supérieure ou égale à 35° C.

11. Utilisation selon l'une quelconque des revendications 1 à 10, selon laquelle le squelette (S) est constitué d'un copolymère obtenu par polymérisation radicalaire :
(a) d'au moins un monomère ou un mélange de monomères (A) à insaturation éthylénique, et
(b) d'au moins un monomère ou un mélange de monomères (B) polaires et hydrophiles, à insaturation éthylénique.

12. Utilisation selon l'une quelconque des revendications 1 à 10, selon laquelle le squelette (S) est constitué d'un copolymère obtenu par polycondensation :
(a) d'au moins un monomère ou un mélange de monomères (A') polycondensables et éventuellement
(b) d'un monomère ou un mélange de monomères (B') polycondensables avec le ou les monomères (A') et portant au moins un groupe fonctionnel hydrophile apportant la solubilité ou la dispersibilité dans l'eau, les milieux alcooliques ou les milieux hydroalcooliques ; les monomères (A') et (B') étant choisis de telle sorte que la température de transition de phase Tg du squelette (S) soit supérieure à 25°C.

13. Utilisation selon la revendication 11, le squelette (S) est un polycondensat choisi dans le groupe constitué par les polyesters, les polyamides, les polyuréthanes ou les polyesteramides.

14. Utilisation selon la revendication 11, selon laquelle les monomères (A) sont choisis par exemple dans le groupe constitué par :
- les esters ou les amides acryliques ou méthacryliques obtenus à partir d'alcools aliphatiques, linéaires, ramifiés ou cycliques et/ou d'alcools aromatiques ;
- les esters ou amides vinyliques, allyliques ou méthallyliques obtenus à partir d'alcools aliphatiques, linéaires, ramifiés ou cycliques et/ou d'alcools aromatiques ;
- les oléfines ;
- les monomères acryliques ou vinyliques fluorés ou perfluorés ainsi que leurs mélanges.

15. Utilisation selon la revendication 11 ou 14, selon laquelle les monomères (A) sont choisis dans le groupe constitué par : le (méth)acrylate de méthyle, le (méth)acrylate d'éthyle, le (méth)acrylate de propyle, le (méth)acrylate de butyle, le (méth)acrylate d'isobutyle, le (méth)acrylate de tertio-butyle, le tertio-butyl acrylamide, l'acétate de vinyle, le propionate de vinyle, le benzoate de vinyle, le tertio-butyl benzoate de vinyle, l'éthylène, le propylène, le styrène ou le styrène substitué ou leurs mélanges.

16. Utilisation selon l'une quelconque des revendications 11, 14 et 15, selon laquelle les monomères (B) sont choisis parmi les monomères à insaturation éthylénique, hydrophiles et polaires, anioniques, cationiques, amphotères ou non-ioniques ou leurs mélanges.

17. Utilisation selon la revendication 16, selon laquelle les monomères (B) anioniques sont choisis dans le groupe constitué par :
- les monomères comportant au moins une fonction acide, sous forme libre ou bien sous forme partiellement ou totalement neutralisée ;
- les monomères comportant au moins une fonction acide sulfonique, sous forme libre ou bien sous forme partiellement ou totalement neutralisée ;
- les monomères comportant au moins une fonction acide phosphorique ou phosphonique, sous forme libre ou bien sous forme partiellement ou totalement neutralisée.

18. Utilisation selon la revendication 17, selon laquelle les monomères (B) anioniques sont choisis parmi les mono acides carboxyliques ; les diacides carboxyliques ou les anhydrides d'acide ainsi que leurs monoesters ou monoamides ; l'acide vinyl- ou styrène sulfonique ; l'acide acrylamido-2 méthylpropane-2 sulfonique, lesdits monomères étant sous forme libre ou bien sous forme partiellement ou totalement neutralisée.

19. Utilisation selon la revendication 16, selon laquelle les monomères (B) cationiques sont choisis dans le groupe constitué par : les monomères comportant au moins une fonction amine sous forme libre ou bien partiellement ou totalement neutralisée ou bien partiellement ou totalement quaternisée.

20. Utilisation selon la revendication 19, selon laquelle les monomères (B) cationiques sont choisis dans le groupe constitué par : le (méth)acrylate de diméthylaminoéthyle, le diméthylaminoéthyl méthacrylamide, le vinylamine, la vinylpyridine, le chlorure de diallyldiméthylammonium, lesdits monomères étant sous forme libre ou bien sous forme partiellement ou totalement neutralisée ou bien sous forme partiellement ou totalement quatemisée.

21. Utilisation selon la revendication 16, selon laquelle les monomères (B) amphotères sont choisis dans le groupe constitué par : les carboxybétaïnes ou les sulfobétaïnes obtenues par quaternisation partielle ou totale de monomères à insaturation éthylénique comportant une fonction amine par des sels de sodium d'acide carboxylique à halogénure mobile ou par des sultones cycliques.

22. Utilisation selon la revendication 16, selon laquelle les monomères (B) non-ioniques sont choisis dans le groupe constitué par :
- les (méth)acrylate d'hydroxyalkyle en C₁-C₄ ;
- les acrylamides ;
- la N-vinylpyrrolidone ;
- les (méth)acrylates d'éthylèneglycol, de diéthylèneglycol, de polyéthylèneglycol à extrémité hydroxyle ou éther.

23. Composition cosmétique ou dermatologique, **caractérisée par le fait qu'**elle contient dans un support cosmétiquement acceptable au moins un copolymère greffé tel que défini selon l'une quelconque des revendications 1 à 22.

24. Composition selon la revendication 23, **caractérisée par le fait que** la concentration en copolymère greffé dans les compositions cosmétiques est généralement comprise entre 0,1 et 50%, et de préférence entre 1 et 30% par rapport au poids total de la composition.

25. Composition selon Ja revendication 23 ou 24, **caractérisée par le fait que** le support cosmétiquement acceptable est de préférence constitué d'eau, d'un ou plusieurs solvants organiques cosmétiquement acceptables ou bien d'un mélange d'eau et d'un ou plusieurs solvants organiques cosmétiquement acceptables.

26. Composition selon la revendication 25, **caractérisée par le fait que** le ou les solvants organiques cosmétiquement acceptables sont choisis parmi les alcools inférieurs en C₁-C₄.

27. Composition selon l'une quelconque des revendications 23 à 26, **caractérisée par le fait que** le copolymère greffé est dissous ou en dispersion dans le support de la composition.

28. Composition selon l'une quelconque des revendications 23 à 27, **caractérisée par le fait qu'**elle contient des additifs cosmétiques conventionnels choisis parmi les corps gras tels que les huiles minérales, végétales animales ou de synthèse, les cires animales, fossiles, végétales, minérales ou de synthèse, des solvants organiques, des agents épaississants, des adoucissants, des agents anti-mousse, des agents hydratants, des humectants, des agents traitants, des antiperspirants, des agents alcanisants, des agents acidifiants, des filtres solaires UV-A ou UV-B ou à bande large, des colorants, des pigments, des parfums, des plastifiants, des conservateurs, des polymères organiques anioniques, non-ioniques ou amphotères et les agents propulseurs

29. Composition selon l'une quelconque des revendications 23 à 28, **caractérisée en ce qu'**il s'agit d'une composition capillaire.

30. Composition selon l'une quelconque des revendications 23 à 29, **caractérisée en ce qu'**il s'agit d'une composition de maquillage.

31. Composition selon l'une quelconque des revendications 23 à 30, **caractérisée en ce qu'**il s'agit d'une composition pour le soin de la peau.

32. Utilisation non-thérapeutique d'un copolymère greffé tel que défini selon l'une quelconque des revendications 1 à 22 comme agent filmogène, ou comme additif d'agent filmogène, dans une composition cosmétique.

33. Procédé de traitement des matières kératiniques, **caractérisé en ce qu'**il consiste à appliquer sur ces dernières une composition telle que définie à l'une quelconque des revendications 23 à 28.

34. Utilisation d'un copolymère greffé tel que défini selon l'une quelconque des revendications 1 à 22 comme agent filmogène, ou comme additif d'agent filmogène, pour la préparation d'une composition dermatologique.

## Claims

1. Use for the preparation of cosmetic or dermatological compositions of a grafted copolymer, the skeleton (S) of which is composed of a hydrophilic copolymer, with a glass transition temperature Tg greater than 25°C, obtained by radical polymerization or by polycondensation, comprising, on the chain of the skeleton (S), at least one graft composed of a hydrophobic macromonomer (M) with a glass transition temperature T'g of less than 25°C.

2. Use according to Claim 1, according to which the grafted macromonomers (M) are chosen from hydrocarbon-comprising, hydrofluorocarbon-comprising or fluorocarbon-comprising hydrophobic macromonomers having a phase transition temperature T'g of less than 25°C.

3. Use according to Claim 1 or 2, according to which the grafted macromonomers (M) have a phase transition temperature T'g of less than 10°C and preferably of less than or equal to 0°C.

4. Use according to any one of Claims 1 to 3, according to which the grafted macromonomers (M) have a surface tension generally of less than or equal to 40 dyne/cm at 20°C.

5. Use according to any one of Claims 1 to 4, according to which the grafted macromonomers (M) have an average molecular weight, measured at the peak tip by steric exclusion chromatography, ranging from 200 to 20,000.

6. Use according to any one of Claims 1 to 5, according to which the grafted macromonomers (M) are chosen from the group consisting of:
(a) polymers and copolymers of linear or branched C₂-C₁₈ alkyl acrylate or methacrylate with a T'g of less than 25°C and exhibiting a terminal group chosen from vinyl, allyl, methallyl, (meth)acryloyl, ethacryloyl, vinylbenzoyl, vinylbenzyl, C₁-C₄ alkenyl or C₁-C₆ cycloalkenyl or a terminal reactive functional group capable of interacting with the skeleton (S) or the monomers constituting it or a terminal reactive functional group capable of participating in a polycondensation;
(b) polyolefins with a T'g of less than 25°C and exhibiting a terminal group possessing ethylenic unsaturation or a terminal reactive functional group capable of interacting with the skeleton (S) or the monomers constituting it or a terminal reactive functional group capable of participating in a polycondensation;
(c) vinyl polymers with a T'g of less than 25°C and exhibiting a terminal group possessing ethylenic unsaturation or a terminal reactive functional group capable of interacting with the skeleton (S) or the monomers constituting it or a terminal reactive functional group capable of participating in a polycondensation;
(d) polymers or copolymers of fluorinated or perfluorinated monomers with a T'g of less than 25°C and exhibiting a terminal group possessing ethylenic unsaturation or a terminal reactive functional group capable of interacting with the skeleton (S) or the monomers constituting it or a terminal reactive functional group capable of participating in a polycondensation;
(e) polyesters with a T'g of less than 25°C and exhibiting a terminal group possessing ethylenic unsaturation or a terminal reactive functional group capable of interacting with the skeleton (S) or the monomers constituting it or a terminal reactive functional group capable of participating in a polycondensation.

7. Use according to Claim 6, according to which the macromonomers are chosen from the group consisting of: poly(butyl acrylate) macromonomers with a monomethacrylate end; poly(butyl acrylate) macromonomers with a monomethacrylate end; poly(2-ethylhexyl acrylate) macromonomers with a monoacrylate or monomethacrylate end; poly(dodecyl acrylate) or poly(dodecyl methacrylate) macromonomers; poly(stearyl acrylate) or poly(stearyl methacrylate) macromonomers; polyethylene macromonomers; polypropylene macromonomers; polyethylene/polypropylene copolymer macromonomers; polyethylene/polybutylene copolymer macromonomers; polybutadiene macromonomers; polyisoprene macromonomers; hydrogenated polybutadiene or hydrogenated polyisoprene macromonomers comprising only 3 or 4 repeat units and more particularly phytol (3,7,11,15-tetramethylhexadec-2-en-1-ol) acrylate or methacrylate; perfluoroalkyl (meth)acrylate homopolymers or copolymers; or aliphatic polyesters composed of long carbonaceous sequences.

8. Use according to any one of Claims 1 to 7, according to which the grafted macromonomers (M) are present in the composition of the copolymers of the invention in a proportion ranging from 1 to 60% by weight with respect to the total weight of the grafted copolymer.

9. Use according to any one of Claims 1 to 7, **characterized in that** the grafted copolymer exhibits an average molecular weight, measured at the peak tip by steric exclusion chromatography, ranging from 10,000 to 5,000,000,

10. Use according to any one of Claims 1 to 8, **characterized in that** the skeleton (S) of the copolymers of the invention has a phase transition temperature Tg of greater than or equal to 35°C.

11. Use according to any one of Claims 1 to 10, according to which the skeleton (S) is composed of a copolymer obtained by radical polymerization:
(a) of at least one monomer or one mixture of monomers (A) possessing ethylenic unsaturation, and
(b) of at least one monomer or one mixture of monomers (B) which are polar and hydrophilic and which possess ethylenic unsaturation.

12. Use according to any one of Claims 1 to 10, according to which the skeleton (S) is composed of a copolymer obtained by polycondensation:
(a) of at least one monomer or one mixture of monomers (A') which are polycondensable and optionally
(b) of a monomer or a mixture of monomers (B'), which are polycondensable with the monomer or monomers (A'), carrying at least one hydrophilic functional group contributing solubility or dispersability in water, alcoholic media or aqueous/alcoholic media, the monomers (A') and (B') being chosen so that the phase transition temperature Tg of the skeleton (S) is greater than 25°C.

13. Use according to Claim 12, according to which the skeleton (S) is a polycondensate chosen from the group consisting of polyesters, polyamides, polyurethanes or polyester amides.

14. Use according to Claim 11, according to which the monomers (A) are chosen, for example, from the group consisting of:
- acrylic or methacrylic esters or amides obtained from linear, branched or cyclic aliphatic alcohols and/or from aromatic alcohols;
- vinyl, allyl or methallyl esters or amides obtained from linear, branched or cyclic aliphatic alcohols and/or from aromatic alcohols;
- olefins;
- fluorinated or perfluorinated acrylic or vinyl monomers, and their mixtures.

15. Use according to Claim 11 or 14, according to which the monomers (A) are chosen from the group consisting of: methyl (meth)acrylate, ethyl (meth)acrylate, propyl (meth)acrylate, butyl (meth)acrylate, isobutyl (meth)acrylate, tert-butyl (meth)acrylate, tert-butylacrylamide, vinyl acetate, vinyl propionate, vinyl benzoate, vinyl tert-butylbenzoate, ethylene, propylene, styrene or substituted styrene, or their mixtures.

16. Use according to any one of Claims 11, 14 and 15, according to which the monomers (B) are chosen from anionic, cationic, amphoteric or non-ionic, hydrophilic and polar monomers possessing ethylenic unsaturation, or their mixtures.

17. Use according to Claim 16, according to which the anionic monomers (B) are chosen from the group consisting of:
- monomers comprising at least one acidic functional group, in the free form or else in the partially or completely neutralized form;
- monomers comprising at least one sulphonic acid functional group, in the free form or else in the partially or completely neutralized form;
- monomers comprising at least one phosphoric or phosphonic acid functional group, in the free form or else in the partially or completely neutralized form.

18. Use according to Claim 17, according to which the anionic monomers (B) are chosen from monocarboxylic acids; dicarboxylic acids or acid anhydrides, as well as their monoesters or monoamides; vinyl- or styrenesulphonic acid; or 2-acrylamido-2-methylpropanesulphonic acid, the said monomers being in the free form or else in the partially or, completely neutralized form.

19. Use according to Claim 16, according to which the cationic monomers (B) are chosen from the group consisting of: monomers comprising at least one amine functional group in the free form or else partially or completely neutralized or else partially or completely quaternized.

20. Use according to Claim 19, according to which the cationic monomers (B) are chosen from the group consisting of: dimethylaminoethyl (meth)acrylate, dimethylaminoethyl-methacrylamide, vinylamine, vinylpyridine or diallyldimethylammonium chloride, the said monomers being in the free form or else in the partially or completely neutralized form or else in the partially or completely quaternized form.

21. Use according to Claim 16, according to which the amphoteric monomers (B) are chosen from the group consisting of: carboxybetaines or sulphobetaines obtained by partial or complete quaternization of monomers possessing ethylenic unsaturation comprising an amine functional group by sodium salts of carboxylic acids possessing a mobile halide or by cyclic sultones.

22. Use according to Claim 16, according to which the non-ionic monomers (B) are chosen from the group consisting of:
- C₁-C₄ hydroxyalkyl (meth)acrylates;
- acrylamides;
- N-vinylpyrrolidone;
- ethylene glycol (meth)acrylate, diethylene glycol (meth)acrylate or (meth)acrylates of polyethylene glycol possessing a hydroxyl or ether end.

23. Cosmetic or dermatological composition, **characterized in that** it contains, in a cosmetically acceptable vehicle, at least one grafted copolymer as defined according to any one of Claims 1 to 22.

24. Composition according to Claim 23, **characterized in that** the concentration of grafted copolymer in the cosmetic compositions is generally between 0.1 and 50% and preferably between 1 and 30% with respect to the total weight of the composition.

25. Composition according to Claim 23 or 24, **characterized in that** the cosmetically acceptable vehicle is preferably composed of water, of one or more cosmetically acceptable organic solvents or else of a mixture of water and of one or more cosmetically acceptable organic solvents.

26. Composition according to Claim 25, **characterized in that** the cosmetically acceptable organic solvent or solvents are chosen from lower C₁-C₄ alcohols.

27. Composition according to any one of Claims 23 to 26, **characterized in that** the grafted copolymer is dissolved or in dispersion in the vehicle for the composition.

28. Composition according to any one of Claims 23 to 27, **characterized in that** it comprises conventional cosmetic additives chosen from fatty substances, such as mineral, vegetable, animal or synthetic oils, animal, fossil, vegetable, mineral or synthetic waxes, organic solvents, thickening agents, softeners, antifoaming agents, moisturizing agents, humectants, treating agents, antiperspirants, basifying agents, acidifying agents, UV-A or UV-B or broad spectrum sunscreens, dyes, pigments, fragrances, plasticizers, preservatives, anionic, non-ionic or amphoteric organic polymers and propellants.

29. Composition according to any one of Claims 23 to 28, **characterized in that** it is a hair composition.

30. Composition according to any one of Claims 23 to 29, **characterized in that** it is a make-up composition.

31. Composition according to any one of Claims 23 to 30, **characterized in that** it is a skin care composition.

32. Non-theraputic use of a grafted copolymer as defined according to any one of Claims 1 to 22 as film-forming agent, or as additive for film-forming agent, in a cosmetic composition.

33. Process for the treatment of keratinous substances, **characterized in that** it consists in applying, to the latter, a composition as defined in any one of Claims 23 to 28.

34. Use of a grafted copolymer as defined according to any one of claims 1 to 22 as film-forming agent, or as additive for film-forming agent, for the preparation of a dermatological composition.

## Patentansprüche

1. Verwendung eines Pfropfcopolymers für die Herstellung von kosmetischen oder dermatologischen Zusammensetzungen, dessen Rückgrat (R) aus einem hydrophilen Copolymer mit einer Glasübergangstemperatur Tg oberhalb von 25 °C besteht, das durch radikalische Polymerisation oder durch Polykondensation erhalten wird und das auf der Kette des Rückgrats mindestens einen Pfropfzweig trägt, der aus einem hydrophoben Makromonomer (M) mit einer Glasübergangstemperatur T'g unterhalb von 25 °C besteht.

2. Verwendung nach Anspruch 1, wobei die aufgepfropften Makromonomere (M) unter den hydrophoben Kohlenwasserstoff-Makromonomeren, den hydrophoben Fluorkohlenwasserstoff-Makromonomeren und den Fluorkohlenstoff-Makromonomeren, die eine Phasenübergangstemperatur T'g unterhalb von 25 °C aufweisen, ausgewählt werden.

3. Verwendung nach Anspruch 1 oder 2, wobei die aufgepfropften Makromonomere (M) eine Phasenübergangstemperatur T'g unterhalb von 10 °C und vorzugsweise von 0°C oder darunter aufweisen.

4. Verwendung nach einem der Ansprüche 1 bis 3, wobei die aufgepfropften Makromonomere (M) bei 20°C eine Oberflächenspannung im allgemeinen von 40 dyne/cm oder darunter aufweisen.

5. Verwendung nach einem der Ansprüche 1 bis 4, wobei die aufgepfropften Makromonomere (M) ein mittleres Molekulargewicht, das bei der Peakspitze durch sterische Ausschlußchromatographie gemessen wird, im Bereich von 200 bis 20000 aufweisen.

6. Verwendung nach einem der Ansprüche 1 bis 5, wobei die gepfropften Makromonomere (M) ausgewählt werden unter:
(a) den geradkettigen oder verzweigten C₂-C₁₈-Alkylacrylat-Polymeren und -Copolymeren und den geradkettigen oder verzweigten C₂-C₁₈-Alkylmethacrylat-Polymeren und -Copolymeren mit T'g unterhalb von 25 °C, die aufweisen: eine endständige Gruppe, die unter Vinyl, Allyl, Methallyl, (Meth)acryloyl, Ethacryloyl, Vinylbenzoyl, Vinylbenzyl, C₁-C₄-Alkenyl, C₁-C₆-Cycloalkenyl ausgewählt wird, oder eine endständige reaktive funktionelle Gruppe, die imstande ist, mit dem Rückgrat (R) oder den Monomeren, aus denen es besteht, in Wechselwirkung zu treten, oder eine endständige reaktive funktionelle Gruppe, die imstande ist, an einer Polykondensation teilzunehmen,
(b) den Polyolefinen mit T'g unter 25 °C, die aufweisen: eine ethylenisch ungesättigte endständige Gruppe oder eine endständige reaktive funktionelle Gruppe, die imstande ist, mit dem Rückgrat (R) oder den Monomeren, aus denen es besteht, in Wechselwirkung zu treten, oder eine endständige reaktive funktionelle Gruppe, die imstande ist, an einer Polykondensation teilzunehmen,
(c) den Vinylpolymeren mit T'g unter 25 °C, die aufweisen: eine ethylenisch ungesättigte endständige Gruppe oder eine endständige reaktive funktionelle Gruppe, die imstande ist, mit dem Rückgrat (R) oder den Monomeren, aus denen es besteht, in Wechselwirkung zu treten, oder eine endständige reaktive funktionelle Gruppe, die imstande ist, an einer Polykondensation teilzunehmen,
(d) den Polymeren oder Copolymeren fluorhaltiger oder perfluorierter Monomere mit T'g unter 25 °C, die aufweisen: eine ethylenisch ungesättigte endständige Gruppe oder eine endständige reaktive funktionelle Gruppe, die imstande ist, mit dem Rückgrat (R) oder den Monomeren, aus denen es besteht, in Wechselwirkung zu treten, oder eine endständige reaktive funktionelle Gruppe, die imstande ist, an einer Polykondensation teilzunehmen,
(e) den Polyestern mit T'g unter 25 °C, die aufweisen: eine ethylenisch ungesättigte endständige Gruppe oder eine endständige reaktive funktionelle Gruppe, die imstande ist, mit dem Rückgrat (R) oder den Monomeren, aus denen es besteht, in Wechselwirkung zu treten, oder eine endständige reaktive funktionelle Gruppe, die imstande ist, an einer Polykondensation teilzunehmen.

7. Verwendung nach Anspruch 6, wobei die Makromonomere ausgewählt werden unter: den Poly(butylacrylat)-Makromonomeren mit Monomethacrylat-Endgruppe; den Poly(2-Ethylhexylacrylat)-Makromonomeren mit Monoacrylat- oder Monomethacrylat-Endgruppe; den Poly(dodecylacrylat)-oder Poly(dodecylmethacrylat)-Makromonomeren; den Poly(stearylacrylat)- oder Poly(stearylmethacrylat)-Makromonomeren; den Polyethylen-Makromonomeren, den Polypropylen-Makromonomeren, den Polyethylen/Polypropylen-Copolymer-Makromonomeren; den Polyethylen/Polybutylen-Copolymer-Makromonomeren; den Polybutadien-Makromonomeren; den Polyisopren-Makromonomeren; den Makromonomeren von hydriertem Polybutadien oder hydriertem Polyisopren, die nur 3 oder 4 wiederkehrende Einheiten enthalten, und vor allem Phytolacrylat oder Phytolmethacrylat (3,7,11,15-Tetramethyl-2-hexadecen-1-ol); den Homopolymeren oder Copolymeren von Perfluoralkyl(meth)acrylat; den aliphatischen Polyestern, die aus langen Kohlenstoffsequenzen bestehen.

8. Verwendung nach einem der Ansprüche 1 bis 7, wobei die aufgepfropften Makromonomere (M) in der Zusammensetzung der erfindungsgemäßen Copolymere in einem Anteil von 1 bis 60 Gew.-%, bezogen auf das Gesamtgewicht des Pfropfcopolymers, enthalten sind.

9. Verwendung nach einem der Ansprüche 1 bis 7, **dadurch gekennzeichnet, daß** das Pfropfcopolymer ein mittleres Molekulargewicht, das bei der Peakspitze durch sterische Ausschlußchromatographie gemessen wird, im Bereich von 10000 bis 5000000 aufweist.

10. Verwendung nach einem der Ansprüche 1 bis 8, **dadurch gekennzeichnet, daß** das Rückgrat (R) der erfindungsgemäßen Copolymere eine Phasenübergangstemperatur Tg von 35 °C oder darüber aufweist.

11. Verwendung nach einem der Ansprüche 1 bis 10, wobei das Rückgrat (R) aus einem Copolymer besteht, das erhalten wird durch radikalische Polymerisation
(a) mindestens eines ethylenisch ungesättigten Monomers (A) oder Gemischs ethylenisch ungesättigter Monomere (A) und
(b) mindestens eines ethylenisch ungesättigten, hydrophilen und polaren Monomers (B) oder Gemischs ethylenisch ungesättigter, hydrophiler und polarer Monomere (B).

12. Verwendung nach einem der Ansprüche 1 bis 10, wobei das Rückgrat (R) aus einem Copolymer besteht, das erhalten wird durch Polykondensation
(a) mindestens eines polykondensierbaren Monomers (A') oder Gemischs polykondensierbarer Monomere (A') und gegebenenfalls
(b) eines Monomers oder eines Gemischs von Monomeren (B'), die mit dem oder den Monomeren (A') polykondensierbar sind und mindestens eine hydrophile funktionelle Gruppe aufweisen, die für die Löslichkeit oder Dispergierbarkeit in Wasser, alkoholischen Medien oder wäßrig-alkoholischen Medien sorgt, wobei die Monomere (A') und (B') so ausgewählt werden, daß die Phasenübergangstemperatur Tg des Rückgrats (R) oberhalb von 25 °C liegt.

13. Verwendung nach Anspruch 11, wobei das Rückgrat (R) ein Polykondensat ist, das unter den Polyestern, den Polyamiden, den Polyurethanen und den Polyesteramiden ausgewählt wird.

14. Verwendung nach Anspruch 11, wobei die Monomere (A) beispielsweise ausgewählt werden unter:
- den Acrylestern, Acrylamiden, Methacrylestern und Methacrylamiden, die ausgehend von geradkettigen, verzweigten oder cyclischen aliphatischen Alkoholen und/oder aromatischen Alkoholen erhalten werden;
- den Vinyl-, Allyl- oder Methallylestern und den Vinyl-, Allyl- oder Methallylamiden, die ausgehend von geradkettigen, verzweigten oder cyclischen aliphatischen Alkoholen und/oder aromatischen Alkoholen erhalten werden;
- den Olefinen;
- den fluorhaltigen oder perfluorierten Acrylmonomeren und den fluorhaltigen oder perfluorierten Vinylmonomeren sowie ihren Gemischen.

15. Verwendung nach Anspruch 11 oder 14, wobei die Monomere (A) ausgewählt werden unter: Methyl(meth)acrylat, Ethyl(meth)acrylat, Propyl(meth)acrylat, Butyl(meth)acrylat, Isobutyl(meth)acrylat, *tert.*-Butyl(meth)acrylat, *tert.*-Butylacrylamid, Vinylacetat, Vinylpropionat Vinylbenzoat, *tert.*-Butylvinylbenzoat, Ethylen, Propylen, Styrol und substituiertem Styrol und ihren Gemischen.

16. Verwendung nach einem der Ansprüche 11, 14 und 15, wobei die Monomere (B) unter den hydrophilen und polaren, anionischen, kationischen, amphoteren oder nichtionischen ethylenisch ungesättigten Monomeren und ihren Gemischen ausgewählt werden.

17. Verwendung nach Anspruch 16, wobei die anionischen Monomere ausgewählt werden unter:
- den Monomeren, die mindestens eine Säurefunktion aufweisen, in freier Form oder in teilweise oder vollständig neutralisierter Form;
- den Monomeren, die mindestens eine Sulfonsäurefunktion aufweisen, in freier Form oder in teilweise oder vollständig neutralisierter Form;
- den Monomeren, die mindestens eine Phosphorsäurefunktion oder Phosphonsäurefunktion aufweisen, in freier Form oder in teilweise oder vollständig neutralisierter Form.

18. Verwendung nach Anspruch 17, wobei die anionischen Monomere (B) unter den Monocarbonsäuren; den Dicarbonsäuren und den Säureanhydriden sowie ihren Monoestern oder Monoamiden; der Vinyl- oder Styrolsulfonsäure, der 2-Acrylamido-2-methylpropansulfonsäure ausgewählt werden, wobei die Monomere in freier Form oder in teilweise oder vollständig neutralisierter Form vorliegen.

19. Verwendung nach Anspruch 16, wobei die kationischen Monomere (B) unter den Monomeren ausgewählt werden, die mindestens eine Amingruppe in freier Form oder in teilweise oder vollständig neutralisierter Form oder in teilweise oder vollständig quaternisierter Form aufweisen.

20. Verwendung nach Anspruch 19, wobei die kationischen Monomere (B) unter Dimethylaminoethyl(meth)acrylat, Dimethylaminoethylmethacrylamid, Vinylamid, Vinylpyridin, Diallyldimethylammoniumchlorid ausgewählt werden, wobei die Monomere in freier Form oder in teilweise oder vollständig neutralisierter Form oder in teilweise oder vollständig quaternisierter Form vorliegen.

21. Verwendung nach Anspruch 16, wobei die amphoteren Monomere (B) unter den Carboxybetainen und den Sulfobetainen ausgewählt werden, die durch teilweise oder vollständige Quaternisierung von ethylenisch ungesättigten Monomeren, die eine Amingruppe aufweisen, mit Natriumsalzen von Carbonsäuren mit beweglichem Halogen oder mit cyclischen Sulftonen erhalten werden.

22. Verwendung nach Anspruch 16, wobei die nichtionischen Monomere ausgewählt werden unter:
- den Hydroxy-C₁-C₄-alkyl(meth)acrylaten;
- den Acrylamiden;
- N-Vinylpyrrolidon;
- Ethylenglykol(meth)acrylat, Diethylenglykol(meth)acrylat, Polyethylenglykol(meth)acrylat mit endständiger Hydroxy- oder Ethergruppe.

23. Kosmetische oder dermatologische Zusammensetzung, **dadurch gekennzeichnet, daß** sie in einem kosmetisch akzeptablen Träger mindestens ein Pfropfcopolymer enthält, das wie in einem der Ansprüche 1 bis 22 definiert ist.

24. Zusammensetzung nach Anspruch 23, **dadurch gekennzeichnet, daß** das Pfropfcopolymer in den kosmetischen Zusammensetzungen im allgemeinen in einer Konzentration im Bereich von 0,1 bis 50 % und vorzugsweise 1 bis 30 %, bezogen auf das Gesamtgewicht der Zusammensetzung, enthalten ist.

25. Zusammensetzung nach Anspruch 23 oder 24, **dadurch gekennzeichnet, daß** der kosmetisch akzeptable Träger vorzugsweise aus Wasser, einem oder mehreren kosmetisch akzeptablen organischen Lösemitteln oder einem Gemisch aus Wasser und einem oder mehreren kosmetisch akzeptablen Lösemitteln besteht.

26. Zusammensetzung nach Anspruch 25, **dadruch gekennzeichnet, daß** das oder die kosmetisch akzeptablen organischen Lösemittel unter den niederen C₁-C₄-Alkoholen ausgewählt sind.

27. Zusammensetzung nach einem der Ansprüche 23 bis 26, **dadurch gekennzeichnet, daß** das Pfropfcopolymer in dem Träger der Zusammensetzung in gelöster Form oder in dispergierter Form enthalten ist.

28. Zusammensetzung nach einem der Ansprüche 23 bis 27, **dadurch gekennzeichnet, daß** sie herkömmliche kosmetische Zusatzstoffe enthält, die ausgewählt sind unter: Fettsubstanzen, wie Mineralölen, pflanzlichen, tierischen oder synthetischen Ölen, tierischen Wachsen, Erdölwachsen, pflanzlichen, mineralischen oder synthetischen Wachsen, organischen Lösemitteln, Verdickungsmitteln, reizlindernden Mitteln, Antischaummitteln, Hydratisierungsmitteln, Behandlungsmitteln, Antihidrotika, alkalisch machenden Mitteln, Säuerungsmitteln, UV-A- oder UV-B- oder Breitbandsonnenschutzfiltern, Farbstoffen, Pigmenten, Parfüms, Weichmachern, Konservierungsmitteln, anionischen, nichtionischen oder amphoteren organischen Polymeren und Treibmitteln.

29. Zusammensetzung nach einem der Ansprüche 23 bis 28, **dadurch gekennzeichnet, daß** es sich um eine Zusammensetzung zur Haarbehandlung handelt.

30. Zusammensetzung nach einem der Ansprüche 23 bis 29, **dadurch gekennzeichnet, daß** es sich um eine Schminkzusammensetzung handelt.

31. Zusammensetzung nach einem der Ansprüche 23 bis 30, **dadurch gekennzeichnet, daß** es sich um eine Zusammensetzung für die Hautpflege handelt.

32. Nicht-therapeutische Verwendung eines Pfropfcopolymers, das wie in einem der Ansprüche 1 bis 22 definiert ist, als Filmbildner oder Zusatzstoff eines Filmbildners in einer kosmetischen Zusammensetzung.

33. Verfahren zur Behandlung von Keratinmaterialien, **dadurch gekennzeichnet, daß** es darin besteht, auf die Keratinmaterialien eine wie in einem der Ansprüche 23 bis 28 definierte Zusammensetzung aufzutragen.

34. Verwendung eines Pfropfcopolymers, das wie in einem der Ansprüche 1 bis 22 definiert ist, als Filmbildner oder Zusatzstoff eines Filmbildners für die Herstellung einer dermatologischen Zusammensetzung.
